# EUROPEAN PATENT APPLICATION

(11) **EP 2 198 828 A2**
(43) Date of publication of application: **23.06.2010**
(21) Application number: 09252851.2
(22) Date of filing: 21.12.2009
(51) Int. Cl.: A61K 8/02, A61K 8/34, A61K 8/44, A61K 8/46, A61Q 5/02, A61Q 19/10

(54) **Dilute structured compositions comprising a branched fatty alcohol**

(30) Priority: 22.12.2008 US 340883
(71) Applicant: Johnson & Johnson Consumer Companies, Inc., Skillman, NJ 08558 (US)
(72) Inventor: Gunn, Euen T., Trenton, NJ 08611-1723 (US); Nystrand, Glenn A., Lebanon, NJ 08833 (US); Fevola, Michael J., Belle Mead, NJ 08502 (US); Sun, Frank C., Branchburg, NJ 08876 (US); Librizzi, Joseph J., Hillsborough, NJ 08844 (US)
(74) Representative: Kirsch, Susan Edith

(57) **Abstract**

Provided are structured compositions comprising a branched fatty alcohol, a surfactant, and at least about 80% water, wherein said composition has a pH of at least about 7, and wherein said composition has DLS₁₀₀₊ intensity of at least about 80%, personal care products comprising such compositions and a method of making a dilute structured composition, comprising combining a branched fatty alcohol and water to form a structured concentrate having a Yield Stress from about 1 Pascal (Pa) to about 1500 Pa; and adding sufficient additional water to form a dilute structured composition having at least about 80% water.

## Description

### FIELD OF INVENTION

The present invention relates to dilute structured compositions comprising at least one branched fatty alcohol, methods of making structured compositions, and uses of such compositions in personal care products.

### DESCRIPTION OF THE RELATED ART

A variety of so-called "structured" compositions for use in personal care, home care, and other consumer products are known in the art. Such structured compositions are often typified by the presence of a lamellar, surfactant-rich phase, and tend to exhibit desirable rheological and aesthetic properties, as well as, significant power to suspend functional ingredients that are not soluble in water.

Applicants have recognized that for certain end uses, including but not limited to certain personal care product applications, it would be desirable to form compositions that have lamellar, surfactant-rich phase that is stable under conditions such as high water concentration and, for example, moderate to high pH, to create a composition that is aesthetically pleasing and/or phase-stable, but may also be capable of retaining or protecting other components of the composition such as a benefit agent. However, applicants have further recognized that the incorporation of a lamellar, surfactant-rich phase into dilute systems tends to be problematic. In particular, applicants have found that that traditional "concentrated" structured systems cannot merely be diluted with water, since this thermodynamically tends to force the lamellar, surfactant-rich phase to disintegrate.

In light of the above, applicants have recognized the need to develop structured compositions that are dilute, and methods of making such compositions.

### SUMMARY OF THE INVENTION

The present invention meets the aforementioned need and overcomes the disadvantages of the prior art. In particular, applicants have discovered that one or more branched fatty alcohols and one or more surfactants can be combined to produce structured compositions that are phase stable under high dilutions.

According to one aspect, the present invention provides a structured composition comprising a branched fatty alcohol, a surfactant, and at least about 80% water, wherein said composition has a pH of at least about 7, preferably a PH of from 7 to 10 and wherein said composition has DLS₁₀₀₊ intensity of at least about 80%.

According to another aspect, a method of making a dilute structured composition, comprises combining a branched fatty alcohol and water to form a structured concentrate having a Yield Stress from about 1 Pascal (Pa) to about 10,000 Pa; and adding sufficient additional water to form a dilute structured composition having at least about 80% water.

According to another aspect the present invention provides a personal care product comprising a composition that comprises a branched fatty alcohol, a surfactant, at least about 80% water, wherein said composition has DLS₁₀₀₊ intensity of at least about 80%. The personal care product further comprises a container for containing said composition.

### DESCRIPTION OF PREFERRED EMBODIMENTS

All percentages listed in this specification are percentages by weight, unless otherwise specifically mentioned.

As used herein the term "dilute structured composition" means a composition that, when tested according to the "Dynamic Light Scattering Test," as described in this specification, has a DLS₁₀₀₊ intensity of at least about 80%.

As used herein the term "structured concentrate," means a composition having a Yield Stress from about 1 Pascal (Pa) to about 10,000 Pa as measured via the "Yield Stress Test" described in the Test Methods below. Examples of certain preferred structured compositions include those having a Yield Stress of from about 1 Pa to about 1500 Pa, preferably about 10 Pa to about 1100 Pa, as measured by the Yield Stress Method described hereafter.

As noted above, applicants have discovered unexpectedly that dilute structured compositions may be obtained by combining at least one branched fatty alcohol with at least one surfactant and at least about 80% water, wherein the composition has a pH of at least about 7.

"Structured" compositions of the present invention may be characterized by including one or more lamellar phases distributed in an exterior phase. By "lamellar phase" it is meant sheet-like structures of hydrophobic moieties essentially sandwiched between hydrophilic moieties. The sheet like structures may be flat or take on curvature.

In certain preferred embodiments, the lamella take on curvature and form enclosed structures or vesicles. The vesicle may be essentially spherulitic, i.e., such as spherulites. In certain other embodiments, the lamella arrange themselves into "multilamellar vesicles," a series of enclosed structures that are concentric or otherwise enclose one another. In other embodiments, the composition may include "worm-like" structures which are essentially structures that are "hybrid" between lamellar sheets and spherulites.

Any of a variety of suitable surfactants may be used in the compositions of the present invention, with anionic surfactants and/or amphoteric surfactants being particularly preferred.

Particularly preferred are structured compositions comprising an anionic surfactant, a betaine and a branched fatty alcohol.

Examples of suitable amphoteric surfactants include betaines such as: alkyl betaines; amidoalkyl betaines; amidoalkyl sultaines; amphophosphates; phosphorylated imidazolines such as phosphobetaines and pyrophosphobetaines, as well as other betaines represented by the following formula:

B-N⁺R₁R₂(CH₂)ₚX⁻

wherein:
B is an alkyl or alkenyl group, preferably a group having from about 7 to about 22 carbon atoms; and
X⁻ is a anionically charged moiety or a neutral (protonated) derivative thereof. As will be recognized by those of skill in the art, the charge on X⁻ may be dependent on the pH of the composition.

Examples of suitable alkyl betaines include those compounds of the formula:

D-N⁺R₉R₁₀(CH₂)ₚCO₂⁻

wherein:
D is an alkyl or alkenyl group having from about 8 to about 22, e.g., from about 8 to about 16 carbon atoms;
R₉ and R₁₀ are each independently an alkyl or hydroxyalkyl group having from about 1 to about 4 carbon atoms; and
p is 1 or 2.

A preferred betaine for use in the present invention is lauryl betaine, available commercially from Huntsman International LLC of The Woodlands, Texas, as "Empigen BB/J."

Examples of suitable amidoalkyl betaines include those compounds of the formula:

F-CO-NH(CH₂)_{q}-N⁺R₁₁R₁₂(CH₂)ₘCO₂⁻

wherein:
F is an alkyl or alkenyl group having from about 7 to about 21, e.g. from about 7 to about 15 carbon atoms;
R₁₁ and R₁₂ are each independently an alkyl or Hydroxyalkyl group having from about 1 to about 4 carbon atoms;
q is an integer from about 2 to about 6; and
m is 1 or 2.

One amidoalkyl betaine is cocamidopropyl betaine, available commercially from Degussa Goldschmidt Chemical Corporation of Hopewell, Virginia under the tradename, "Tegobetaine L7."

Examples of suitable amidoalkyl sultaines include those compounds of the formula 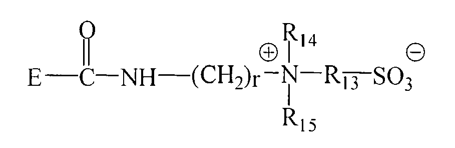 wherein:
E is an alkyl or alkenyl group having from about 7 to about 21, e.g. from about 7 to about 15 carbon atoms;
R₁₄ and R₁₅ are each independently an alkyl, or hydroxyalkyl group having from about 1 to about 4 carbon atoms;
r is an integer from about 2 to about 6; and
R₁₃ is an alkylene or hydroxyalkylene group having from about 2 to about 3 carbon atoms;

The amidoalkyl sultaine may suitably be cocamidopropyl hydroxysultaine, available commercially from Rhodia Inc. of Cranbury, New Jersey under the tradename, "Mirataine CBS."

Examples of suitable amphophosphates compounds include those of the formula: 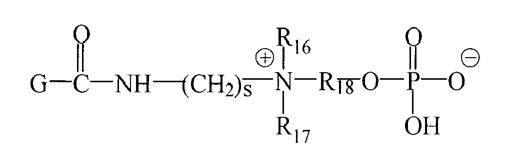 wherein
G is an alkyl or alkenyl group having about 7 to about 21, e.g. from about 7 to about 15 carbon atoms;
s is an integer from about 2 to about 6;
R₁₆ is hydrogen or a carboxyalkyl group containing from about 2 to about 3 carbon atoms;
R₁₇ is a hydroxyalkyl group containing from about 2 to about 3 carbon atoms or a group of the formula:

R₁₉-O-(CH₂)ₜCO₂⁻

wherein
R₁₉ is an alkylene or hydroxyalkylene group having from about 2 to about 3 carbon atoms; and
t is 1 or 2; and
R₁₈ is an alkylene or hydroxyalkylene group having from about 2 to about 3 carbon atoms.

The amphophosphate compounds may suitably be sodium lauroampho PG-acetate phosphate, available commercially from Uniqema of Chicago, Illinois under the tradename, "Monateric 1023," and those disclosed in U.S. Patent 4,380,637, which is incorporated herein by reference.

Examples of suitable phosphobetaines include those compounds of the formula: 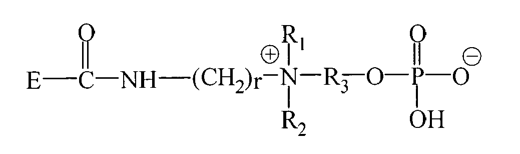 wherein
E, r, R₁, R₂ and R₃, are as defined above.

In one embodiment, the phosphobetaine compounds are those disclosed in U.S. Patent Nos. 4,215,064, 4,617,414, and 4,233,192, which are all incorporated herein by reference.

Examples of suitable pyrophosphobetaines include those compounds of the formula: 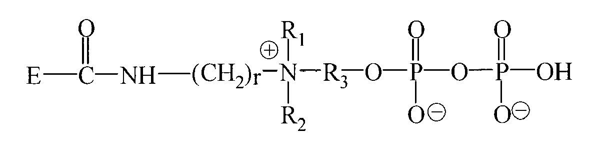 wherein
E, r, R₁, R₂ and R₃, are as defined above.

In one embodiment, the pyrophosphobetaine compounds are those disclosed in U.S. Patent Nos. 4,382,036, 4,372,869, and 4,617,414, which are all incorporated herein by reference.

Examples of other amphoteric surfactants that may be suitable for use in the present invention include, but are not limited to amphocarboxylates such as alkylamphoacetates (mono or di); phosphorylated imidazolines such as phosphobetaines and pyrophosphobetaines; carboxyalkyl alkyl polyamines; alkylimino-dipropionates; alkylamphoglycinates (mono or di); alkylamphoproprionates (mono or di),); N-alkyl β-aminoproprionic acids; alkylpolyamino carboxylates; and mixtures thereof.

Examples of suitable amphocarboxylate compounds include those of the formula:

A-CONH(CH₂)ₓN⁺R₅R₆R₇

wherein
A is an alkyl or alkenyl group having from about 7 to about 21, e.g. from about 10 to about 16 carbon atoms;
x is an integer of from about 2 to about 6;
R₅ is hydrogen or a carboxyalkyl group containing from about 2 to about 3 carbon atoms;
R₆ is a hydroxyalkyl group containing from about 2 to about 3 carbon atoms or is a group of the formula:

R₈-O-(CH₂)ₙCO₂⁻

wherein
R₈ is an alkylene group having from about 2 to about 3 carbon atoms; and
n is 1 or 2; and
R₇ is a carboxyalkyl group containing from about 2 to about 3 carbon atoms.

Examples of suitable amphophosphate compounds include those of the formula: 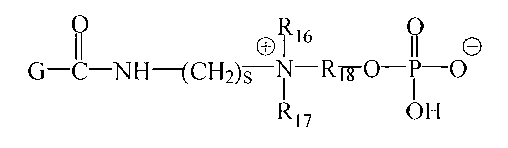 wherein
G is an alkyl or alkenyl group having about 7 to about 21, e.g. from about 7 to about 15 carbon atoms;
s is an integer from about 2 to about 6;
R₁₆ is hydrogen or a carboxyalkyl group containing from about 2 to about 3 carbon atoms;
R₁₇ is a hydroxyalkyl group containing from about 2 to about 3 carbon atoms or a group of the formula:

R₁₉-O-(CH₂)ₜ-CO₂⁻

wherein
R₁₉ is an alkylene or hydroxyalkylene group having from about 2 to about 3 carbon atoms; and
t is 1 or 2; and
R₁₈ is an alkylene or hydroxyalkylene group having from about 2 to about 3 carbon atoms.

Suitable amphophosphate compounds are sodium lauroampho PG-acetate phosphate, available commercially from Uniqema of Chicago, Illinois under the tradename, "Monateric 1023," and those disclosed in U.S. Patent 4,380,637, which is incorporated herein by reference.

Examples of suitable carboxyalkyl alkylpolyamines include those of the formula: 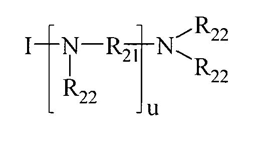 wherein
I is an alkyl or alkenyl group containing from about 8 to about 22, e.g. from about 8 to about 16 carbon atoms;
R₂₂ is a carboxyalkyl group having from about 2 to about 3 carbon atoms;
R₂₁ is an alkylene group having from about 2 to about 3 carbon atoms and
u is an integer from about 1 to about 4.

In order to provide a high degree of cost-effectiveness, the weight fraction of betaine relative to all amphoteric surfactants in the composition may be at least about 25%, preferably at least about 50%, and most preferably at least about 75%.

The dilute structured composition may include any of a variety of suitable anionic surfactants. Suitable anionic surfactants may be branched or unbranched and may include alkyl sulfates, alkyl ether sulfates, alkyl monoglyceryl ether sulfates, alkyl sulfonates, alkylaryl sulfonates, alkyl sulfosuccinates, alkyl ether sulfosuccinates, alkyl sulfosuccinamates, alkyl amidosulfosuccinates, alkyl carboxylates, alkyl amidoethercarboxylates, alkyl succinates, fatty acyl sarcosinates, fatty acyl amino acids, fatty acyl taurates, fatty alkyl sulfoacetates, alkyl phosphates, and mixtures of two or more thereof. Examples of certain anionic surfactants include:
alkyl sulfates of the formula

   R'-CH₂OSO₃X';
alkyl ether sulfates of the formula

   R'(OCH₂CH₂)ᵥOSO₃X';
alkyl monoglyceryl ether sulfates of the formula 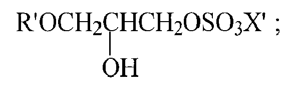
alkyl monoglyceride sulfates of the formula 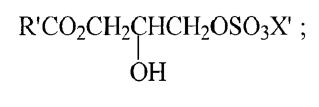
alkyl monoglyceride sulfonates of the formula 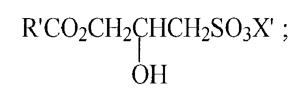
alkyl sulfonates of the formula

   R'-SO₃X';
alkylaryl sulfonates of the formula 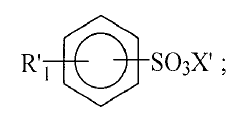
alkyl sulfosuccinates of the formula: 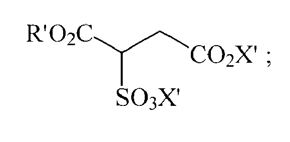
alkyl ether sulfosuccinates of the formula: 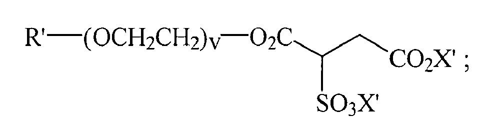
alkyl sulfosuccinamates of the formula: 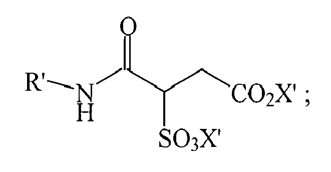
alkyl amidosulfosuccinates of the formula 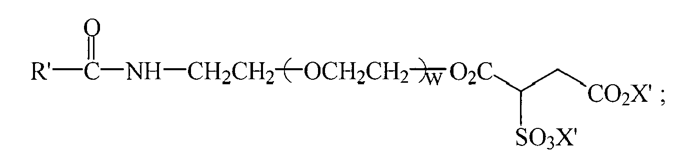
alkyl carboxylates of the formula:

   R'-(OCH₂CH₂)_{w}-OCH₂CO₂X';
alkyl amidoethercarboxylates of the formula: 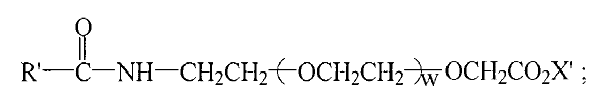
alkyl succinates of the formula: 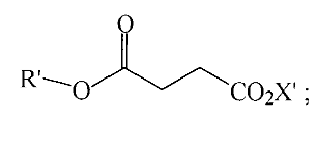
fatty acyl sarcosinates of the formula: 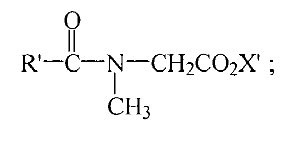
fatty acyl amino acids of the formula: 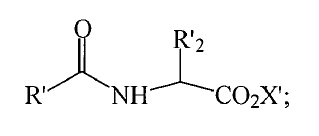
fatty acyl taurates of the formula: 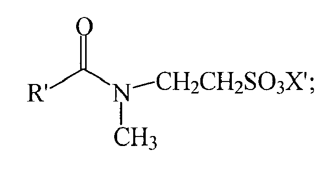
fatty alkyl sulfoacetates of the formula: 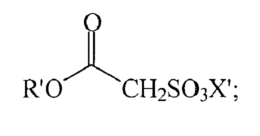
alkyl phosphates of the formula: 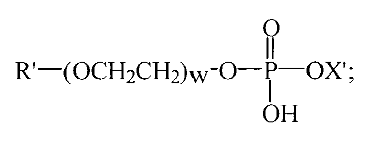
wherein
R' is an alkyl group having from about 7 to about 22, and preferably from about 7 to about 16 carbon atoms,
R'₁ is an alkyl group having from about 1 to about 18, and preferably from about 8 to about 14 carbon atoms,
R'₂ is a substituent of a natural or synthetic I-amino acid,
X' is selected from the group consisting of alkali metal ions, alkaline earth metal ions, ammonium ions, and ammonium ions substituted with from about 1 to about 3 substituents, each of the substituents may be the same or different and are selected from the group consisting of alkyl groups having from 1 to 4 carbon atoms and hydroxyalkyl groups having from about 2 to about 4 carbon atoms and
v is an integer from 1 to 6;
w is an integer from 0 to 20;
and mixtures thereof.

Preferably, the anionic surfactant for use in the present invention comprises a branched anionic surfactant. By "branched anionic surfactant," it is meant an anionic surfactant comprising more than 10% branched surfactant molecules. Suitable branched anionic surfactants include tridecanol based sulfates such as sodium trideceth sulfate, which generally comprises a high level of branching, with over 80% of surfactant molecules comprising at least 2 branches. Another suitable branched anionic surfactant is a C₁₂₋₁₃ alkyl sulfate derived from SAFOL 23 alcohol (Sasol, Inc, Houston, Tex., USA) which has about 15-30% branched surfactant molecules.

Branched anionic surfactants include but are not limited to the following branched anionic alkyl sulfate or alkyl ether sulfate surfactants: sodium tridecyl sulfate, sodium C₁₂₋₁₃ alkyl sulfate, sodium C₁₂₋₁₅ alkyl sulfate, sodium C₁₂₋₁₅ alkyl sulfate, sodium C₁₂₋₁₈ alkyl sulfate, sodium C₁₀₋₁₆ alkyl sulfate, sodium trideceth sulfate, sodium C₁₂-₁₃ pareth sulfate, sodium C₁₂-₁₃ pareth-n sulfate, and sodium C₁₂₋₁₄ pareth-n sulfate. One particularly suitable branched anionic surfactant (about 50% branched) is a sodium trideceth sulfate, available as CEDEPAL TD 430 MFLD from Stepan Company of Northfield, Illinois.

Other salts of all the aforementioned branched anionic surfactants are useful, such as TEA, DEA, ammonia, potassium salts. Useful alkoxylates include the ethylene oxide, propylene oxide and EO/PO mixed alkoxylates. Phosphates, carboxylates and sulfonates prepared from branched alcohols are also useful anionic branched surfactants. Branched anonic surfactants can be derived from synthetic alcohols such as the primary alcohols from the liquid hydrocarbons produced by Fischer-Tropsch condensed syngas, for example SAFOL 23 Alcohol available from Sasol North America, Houston, TX; from synthetically made alcohols such as those described in U.S. Pat. No. 6,335,312 issued to Coffindaffer, et al on Jan. 1, 2002. Preferred alcohols are SAFOL.TM. 23. Preferred alkoxylated alcohols are SAFOL 23-3. Sulfates can be prepared by conventional processes to high purity from a sulfur based SO₃ air stream process, chlorosulfonic acid process, sulfuric acid process, or Oleum process. Preparation via SO₃ air stream in a falling film reactor is a preferred sulfation process.

Suitable branched anionic surfactants include but are not limited to the branched anionic sulfates derived from SAFOL 23-n as previously described, where n is an integer between 1 and about 20. Fractional alkloxylation is also useful, for example by stoichiometrically adding only about 0.3 moles EO, or 1.5 moles EO, or 2.2 moles EO, based on the moles of alcohol present, since the molecular combinations that result are in fact always distributions of alkoxylates so that representation of n as an integer is merely an average representation. Preferred monomethyl branched anionic surfactants include a C₁₂₋₁₃ alkyl sulfate derived from the sulfation of SAFOL 23, which has about 28% branched anionic surfactant molecules.

When the branched anionic surfactant is a branched anionic primary sulfate, it may contain some of the following branched anionic surfactant molecules: 4-methyl undecyl sulfate, 5-methyl undecyl sulfate, 7-methyl undecyl sulfate, 8-methyl undecyl sulfate, 7-methyl dodecyl sulfate, 8-methyl-dodecyl sulfate, 9-methyl dodecyl sulfate,4,5-dimethyl decyl sulfate, 6,9-dimethyl decyl sulfate, 6,9-dimethyl undecyl sulfate, 5-methyl-8-ethyl undecyl sulfate, 9-methyl undecyl sulfate, 5,6,8-trimethyl decyl sulfate, 2-methyl dodecyl sulfate, and 2-methyl undecyl sulfate. When the anionic surfactant is a primary alkoxylated sulfate, these same molecules may be present as the n=0 unreacted alcohol sulfates, in addition to the typical alkoxylated adducts that result from alkoxylation.

Any amount less than about 20% of the one or more surfactants described above may suitable for use in the dilute structured composition, in conjunction with other ingredients in the composition, to produce the dilute structured composition, in accord with the invention. One or more surfactants may suitably be used in a concentration from greater than about 0.1 % to about 20% by weight of active surfactant in the composition. Preferably, one or more surfactants are in present in a concentration from about 0.1% to about 10%, more preferably from about 0.1 % to about 5%, even more preferably from about 0.1 % to about 1%, even more preferably from about 0.2% to about 0.5% of active total surfactant in the composition.

In one particularly preferred embodiment, the dilute structured composition includes both a betaine and branched anionic surfactant. The betaine and the branched anionic surfactant may be present in a betaine to branched anionic surfactant ratio (weight to weight on an actives basis) that is from about 0.1 to about 10:1, preferably from about 0.25:1 to about 4:1, more preferably from about 0.5:1 to about 2:1.

Any of a variety of suitable branched fatty alcohols may be used in the present invention. By "branched fatty alcohol", it is meant, any of various alcohols derived from oils and fats (e.g., from plant or animal sources) or synthetic hydrophobic groups having at least one pendant hydrocarbon-comprising chain. The branched fatty alcohol may comprise any number of carbon atoms, such as from about 8 to about 34, preferably from about 7 to about 22 carbon atoms, more preferably about 9 to about 16 carbon atoms, and even more preferably about 11 to about 16 carbon atoms. Suitable branched fatty alcohols may comprise one or more alcohol groups per molecule. In certain preferred embodiments, the fatty alcohol comprises one alcohol group per molecule.

Suitable branched fatty alcohols may comprise one or more branches in the carbon backbone of the molecule. In certain preferred embodiments, the branched fatty alcohol is monobranched. By "monobranched", it is meant the fatty alcohol has an alkyl chain with one (CH) functional group resulting in one branch in the alkyl chain, i.e. the fatty alcohol has one and only one carbon that has one hydrogen atom and three carbon atoms bonded thereto.

The branched fatty alcohol may be a primary alcohol. By "primary alcohol," it is meant no -COH group is bonded to more than one carbon atom.

Preferably, the branched fatty alcohol is both monobranched and a primary alcohol. In a more particularly preferred embodiment, the branched fatty alcohol is both monobranched and a primary alcohol and has only one alcohol group per molecule.

The branched fatty alcohol may suitably consist solely of hydrogen, carbon, and oxygen atoms. The carbon-carbon bonds within the branched fatty alcohol may be saturated or unsaturated.

In one particularly preferred embodiment, the branched fatty alcohol is a monobranched primary fatty alcohol that can be represented by the following structure (I): 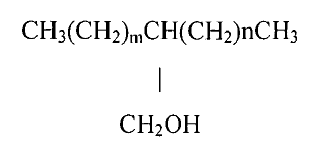 wherein:
m + n = 8 to 27 (inclusive);
m is an integer that ranges from 0 to 14 (inclusive); and
n is an integer that ranges from 0 to 11 (inclusive).

Commercially available materials that are particularly suitable for use as the branched fatty alcohol include the following materials alone or in combination: Isalchem 123, Isofol 28, or Lialchem 123 produced by Sasol Chemical Co of Bad Homburg, Germany. In a particularly preferred embodiment, the branched fatty alcohol is Isofol 28, also known as "2-dodecylhexadecanol." Using the structural nomenclature (I) above, for 2-dodecylhexadecanol, m = 14, n =11, m+n= 25. Alternatively, 2-dodecylhexadecanol can be expressed as the structure (II) below where R¹ is C₁₄H₂₅, and R² is C₁₂H₂₅.

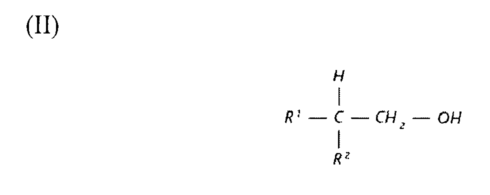

The branched fatty alcohol may be a so-called "Guerbert alcohol," i.e., an alcohol that is fomed by converting a primary aliphatic alcohol into its β-alkylated dimer alcohol with loss of one equivalent of water. This may be particularly suitable for forming branched fatty alcohol from naturally derived fat or oil. One suitable example of a Guerber alcohol is Isofol 20 (octyl-2-dodeacnol) also available from Sasol. Using the structural nomenclature (I) above, for (octyl-2-dodeacnol), m = 9, n =7, m+n=16. Alternatively, (octyl-2-dodeacnol) can be expressed using structure (II) for Isofol 28, except R¹ is C₁₀H₂₅, and R² is C₈H₁₇.

In another embodiment, the branched fatty alcohol includes an alkoxylate moiety, such as ethoxy and/or propoxy groups. Any number of alkoxy groups are acceptable as long as the fatty alcohol is still capable of providing a structured composition. In one embodiment, the fatty alcohol has up to an including 10 alkoxy groups, more preferably from 0 to 3 alkoxy groups, most preferably from 1 to 3 alkoxy groups. Other suitable branched fatty alcohols include cis 2 unsaturated fatty alcohols.

The total concentration of the branched fatty alcohols in the dilute structured composition of the invention is preferably from about 0.01 % to about 5% by weight of active branched fatty alcohol in the composition, more preferably from 0.025% to about 1% by weight, even more preferably from about 0.05% to about 0.25%. By "total concentration of branched fatty alcohols" it is meant the sum of the concentrations of all branched fatty alcohols present in the composition.

In one embodiment of the invention, the branched fatty alcohol and betaine are present in a fatty alcohol to betaine (weight to weight, on an actives basis) ratio that is from about 0.15:1 to about 0.35:1.

In addition to the branched fatty alcohol, to enhance the structuring or provide other benefits, the composition may further include a fatty acid, e.g., cis-2 decenoic acid and 2-alpha, beta unsaturated fatty acids.

Additional surfactants, such as cationic, non-ionic, or combinations thereof may be used in compositions of the present invention. For example, any of a variety of amphoteric surfactants are suitable for use in the present invention. The amphoteric surfactants are disclosed herein without a counter ion. One skilled in the art would readily recognize that under the pH conditions of the compositions of the present invention, the amphoteric surfactants are either electrically neutral by virtue of having balancing positive and negative charges, or they have counter ions such as alkali metal, alkaline earth, or ammonium counter ions.

Various nonionic surfactants may also be suitable. Examples of suitable nonionic surfactants include, but are not limited to, fatty alcohol acid or amide ethoxylates, monoglyceride ethoxylates, sorbitan ester ethoxylates alkyl polyglycosides, mixtures thereof, and the like. Certain preferred nonionic surfactants include polyoxyethylene derivatives of polyol esters, wherein the polyoxyethylene derivative of polyol ester (1) is derived from (a) a fatty acid containing from about 8 to about 22, and preferably from about 10 to about 14 carbon atoms, and (b) a polyol selected from sorbitol, sorbitan, glucose, α-methyl glucoside, polyglucose having an average of about 1 to about 3 glucose residues per molecule, glycerine, pentaerythritol and mixtures thereof, (2) contains an average of from about 10 to about 120, and preferably about 20 to about 80 oxyethylene units; and (3) has an average of about 1 to about 3 fatty acid residues per mole of polyoxyethylene derivative of polyol ester. Examples of such preferred polyoxyethylene derivatives of polyol esters include, but are not limited to PEG-80 sorbitan laurate and Polysorbate 20. PEG-80 sorbitan laurate, which is a sorbitan monoester of lauric acid ethoxylated with an average of about 80 moles of ethylene oxide, is available commercially from Uniqema of Chicago, Illinois under the tradename, "Atlas G-4280." Polysorbate 20, which is the laurate monoester of a mixture of sorbitol and sorbitol anhydrides condensed with approximately 20 moles of ethylene oxide, is available commercially from ICI Surfactants of Wilmington, Delaware under the tradename "Tween 20."

Another class of suitable nonionic surfactants includes long chain alkyl glucosides or polyglucosides, which are the condensation products of (a) a long chain alcohol containing from about 6 to about 22, and preferably from about 8 to about 14 carbon atoms, with (b) glucose or a glucose-containing polymer. Preferred alkyl gluocosides comprise from about 1 to about 6 glucose residues per molecule of alkyl glucoside. A preferred glucoside is decyl glucoside, which is the condensation product of decyl alcohol with a glucose polymer and is available commercially from Cognis Corporation of Ambler, Pennsylvania under the tradename, "Plantaren 2000."

Various cationic surfactants may also be suitable for use in the present compositions. Examples of suitable cationic surfactants include, but are not limited to alkyl quaternaries, benzyl quaternaries, ester quaternaries, ethoxylated quaternaries, alkyl amines, and mixtures thereof, wherein the alkyl group has from about 6 carbon atoms to about 30 carbon atoms, with about 8 to about 22 carbon atoms being preferred.

Any amounts of nonionic surfactant and/or cationic surfactants suitable to produce a structured composition may be combined according to the present methods. For example, the amount of nonionic surfactant and/or cationic surfactants used in the present invention may be from about 0.05% to about 1%, more preferably from about 0.1% to about 0.5%.

As will be recognized by those of skill in the art, the compositions of the present invention further comprise water, which serves to provide a vehicle about which a structured phase is dispersed. The concentration of water is at least about 80% by weight. The concentration of water may be at least about 85%, preferably from about 85% to about 99.5%, more preferably from about 90% to about 99%, and most preferably from about 95% to about 99%.

Compositions of the present invention may include other functional ingredients. By other functional ingredients it is meant any moiety that serves one or more functions either to stabilize or provide aesthetic benefits to the composition or to impart one or more of various benefits to the end user. These various functional ingredients may be of any form at room temperature (e.g., solids, liquids, pastes and the like) and be dispersed, emulsified, or solubilized or otherwise homogenized within the composition.

A wide variety of functional ingredients may be used in compositions of the present invention, although it is preferred that the ingredient does not adversely affect the phase stability of the composition. By "adversely effect the phase stability," it is meant that by including the particular functional ingredient, when subject to a stability challenge (e.g., held at 22° C., 50% relative humidity for a week; when subject to three 48 hour freeze-thaw cycles) the composition irrevocably phase separates into two or more visually distinct phases so as to be displeasing (e.g., in a tactile, olfactory, and/or visual sense) for topical use.

Functional ingredients that may be used include, but are in no way limited to: dyes and colorants; ultraviolet filters and suncsreens, opacificiers, matting agents, rheology modifiers, oils, emollients, and skin conditioners; chelating and sequestering agents, pH adjusters, humectants, film forming polymers, plasticizers, fragrance components; water soluble solvents such as glycols including glycerol, propylene glycol C₁-C₆ alcohols may be incorporated into the composition (again, as long as There is no adverse effect on phase stability) and various benefit agents, as described below.

The functional ingredient may be water-insoluble. By "water-insoluble," it is meant, a moiety that cannot be rendered essentially completely soluble in deionized water at 25°C, after providing a I % by weight of said moiety in said deionized water under moderate agitation for 10 minutes. A wide variety of water-insoluble components may be incorporated into compositions of the present invention. The structured nature of the composition is suitable for dispersing water insoluble components that are solid at room temperature (e.g., certain polymers and waxes; dyes; and particulates such as mineral oxides, silicates, aluminosilicates, zinc pyrithione, colloidal oat flour, soy derivatives and the like) or liquid at room temperature (e.g., oils, emollients, and skin conditioners; biological actives; fragrance components).

By way of example, any of a variety of commercially available pearlescent or opacifying agents which are capable of suspending water insoluble additives such as silicones and/or which tend to indicate to consumers that the resultant product is a conditioning shampoo are suitable for use in this invention. The pearlescent or opacifying agent may be present in an amount, based upon the total weight of the composition, of from about 0.01% percent to about 0.1 % percent. Examples of suitable pearlescent or opacifying agents include, but are not limited to mono or diesters of (a) fatty acids having from about 16 to about 22 carbon atoms and (b) either ethylene or propylene glycol; mono or diesters of (a) fatty acids having from about 16 to about 22 carbon atoms (b) a polyalkylene glycol of the formula: HO-(JO)ₐ-H, wherein J is an alkylene group having from about 2 to about 3 carbon atoms; and a is 2 or 3;fatty alcohols containing from about 16 to about 22 carbon atoms; fatty esters of the formula: KCOOCH₂L, wherein K and L independently contain from about 15 to about 21 carbon atoms; inorganic solids insoluble in the shampoo composition, and mixtures thereof

The pearlescent or opacifying agent may be introduced to the structured composition as a pre-formed, stabilized aqueous dispersion, such as that commercially available from Cognis Corporation of Ambler, Pennsylvania under the tradename, "Euperlan PK-3000." This material is a combination of glycol distearate (the diester of ethylene glycol and stearic acid), Laureth-4 (CH₃(CH₂)₁₀CH₂(OCH₂CH₂)₄OH) and cocamidopropyl betaine and may be in a weight percent ratio of from about 25 to about 30: about 3 to about 15: about 20 to about 25, respectively.

Any of a variety of commercially available secondary conditioners, such as volatile silicones, which impart additional attributes, such as gloss to the hair are suitable for use in this invention. The volatile silicone conditioning agent has an atmospheric pressure boiling point less than about 220°C. The volatile silicone conditioner may be present in an amount of from about 0 percent to about 3 percent, e.g. from about 0.05 percent to about 1 percent, based on the overall weight of the composition. Examples of suitable volatile silicones nonexclusively include polydimethylsiloxane, polydimethylcyclosiloxane, hexamethyldisiloxane, cyclomethicone fluids such as polydimethylcyclosiloxane available commercially from Dow Coming Corporation of Midland, Michigan under the tradename, "DC-345" and mixtures thereof, and preferably include cyclomethicone fluids. Other suitable secondary conditioners include cationic polymers, including polyquarterniums, cationic guar, and the like.

Any of a variety of commercially available humectants, which are capable of providing moisturization and conditioning properties to the personal cleansing composition, are suitable for use in the present invention. The humectant may be present in an amount of from about 0 percent to about 10 percent, e.g. from about 0.05 percent to about 0.5 percent, based on the overall weight of the composition. Examples of suitable humectants nonexclusively include: 1) water soluble liquid polyols selected from the group comprising glycerine, propylene glycol, hexylene glycol, butylene glycol, dipropylene glycol, polyglycerols, and mixtures thereof; 2)polyalkylene glycol of the formula: HO-(R"O)_{b}-H, wherein R" is an alkylene group having from about 2 to about 3 carbon atoms and b is an integer of from about 2 to about 10; 3) polyethylene glycol ether of methyl glucose of formula CH₃-C₆H₁₀O₅-(OCH₂CH₂)_{c}-OH, wherein c is an integer from about 5 to about 25; 4) urea; and 5) mixtures thereof, with glycerine being the preferred humectant.

Examples of suitable chelating agents include those which are capable of protecting and preserving the compositions of this invention. Preferably, the chelating agent is ethylenediamine tetracetic acid ("EDTA"), and more preferably is tetrasodium EDTA, available commercially from Dow Chemical Company of Midland, Michigan under the tradename, "Versene 100XL" and is present in an amount, based upon the total weight of the composition, from about 0 to about 0.5 percent or from about 0.05 percent to about 0.25 percent.

Suitable preservatives include, for example, parabens, quaternary ammonium species, phenoxyethanol, benzoates, DMDM hydantoin, and are present in the composition in an amount, based upon the total weight of the composition, from about 0 to about 0.2 percent or from about 0.005 percent to about 0.05 percent.

It also may be desirable to incorporate any of a variety of commercially available thickening agents, which are capable of imparting the appropriate viscosity to the personal care compositions are suitable for use in this invention.

Examples of suitable thickening agents nonexclusively include: mono or diesters of 1) polyethylene glycol of formula: HO-(CH₂CH₂O)_{z}H, wherein z is an integer from about 3 to about 200; and 2) fatty acids containing from about 16 to about 22 carbon atoms; fatty acid esters of ethoxylated polyols; ethoxylated derivatives of mono and diesters of fatty acids and glycerine; hydroxyalkyl cellulose; alkyl cellulose; hydroxyalkyl alkyl cellulose; hydrophobically-moditied alkali swellable emulsions (HASEs); hydrophobically-modified ethoxylated urethanes (HEURs); xantham and guar gums; and mixtures thereof. Preferred thickeners include polyethylene glycol ester, and more preferably PEG-150 distearate which is available from the Stepan Company of Northfield, Illinois or from Comiel, S.p.A. of Bologna, Italy under the tradename, "PEG 6000 DS". Thickening agents may be present in the composition in an amount sufficient to achieve a desirable viscosity or other rheological characteristics. The concentration of the thickener, based upon the total weight of the composition, from about 0 to about 1 percent, such as from about 0.005 percent to about 0.5 percent.

Compositions of the present invention may include a benefit agent. A benefit agent is any element, an ion, a compound (e.g., a synthetic compound or a compound isolated from a natural source) or other chemical moiety in solid (e.g. particulate), liquid, or gaseous state and compound that has a cosmetic or therapeutic effect on the skin, hair, mucosa, or teeth. As used herein, the term "benefit agent" includes any active ingredient such as a cosmetic or pharmaceutical, that is to be delivered into and/or onto the skin, hair, mucosa, or teeth at a desired location.

The benefit agents useful herein may be categorized by their therapeutic benefit or their postulated mode of action. However, it is to be understood that the benefit agents useful herein may, in some circumstances, provide more than one therapeutic benefit or operate via greater than one mode of action. Therefore, the particular classifications provided herein are made for the sake of convenience and are not intended to limit the benefit agents to the particular application(s) listed.

Examples of suitable benefit agents include those that provide benefits such as, but not limited to: emollients, moisturizing and water-loss prevention agents; cleansing agents; depigmentation agents; reflectants and optical modifiers; amino acids and their derivatives; antimicrobial agents; allergy inhibitors; anti-acne agents; anti-aging agents; anti-wrinkling agents, antiseptics; analgesics; shine-control agents; antipruritics; local anesthetics; anti-hair loss agents; hair growth promoting agents; hair growth inhibitor agents, antihistamines; antiinfectives; anti-inflammatory agents; anticholinergics; vasoconstrictors; vasodilators; wound healing promoters; peptides, polypeptides and proteins; deodorants and anti-perspirants; medicament agents; skin firming agents, vitamins; skin lightening agents; skin darkening agents; antifungals; depilating agents; counterirritants; hemorrhoidals; insecticides; enzymes for exfoliation or other functional benefits; enzyme inhibitors; poison ivy products; poison oak products; burn products; anti-diaper rash agents; prickly heat agents; vitamins; herbal extracts; vitamin A and its derivatives; flavenoids; sensates and stress-reducing agents; anti-oxidants; hair lighteners; sunscreens; anti-edema agents, neo-collagen enhancers, antidandruff/sebhorreic dermatitis/psoriasis agents; keratolytics; lubricants; lightening and whitening agents; calcification, fluoridation and mineralization agents; and mixtures thereof.

The amount of the benefit agent that may be used may vary depending upon, for example, the ability of the benefit agent to penetrate through the skin, nail, mucosa, or teeth; the specific benefit agent chosen, the particular benefit desired, the sensitivity of the user to the benefit agent, the health condition, age, and skin and/or nail condition of the user, and the like. In sum, the benefit agent is used in a "safe and effective amount," which is an amount that is high enough to deliver a desired skin or nail benefit or to modify a certain condition to be treated, but is low enough to avoid serious side effects, at a reasonable risk to benefit ratio within the scope of sound medical judgment.

Compositions of the present invention are structured, i.e., preferably include a lamellar phase that is largely composed of one or more surfactants that is dispersed within an exterior (typically aqueous) phase. The viscosity of the personal care composition may vary from essentially water thin to a spreadable such as that of a cream or lotion or gel. For example, when measured using a LVT3 spindle at 30 rpm, the viscosity may be from about 1 cps to about 2000cps.

The pH of the present dilute structured compositions may be in a range from about 5 to about 10. However, the inventors have found that lamellar phase present in the composition may be particularly durable when the dilute structured composition has a pH that is from about 7 to about 10.

Compositions of the present invention are typically extrudable or dispensable from a container or package, such as to be applied directly or indirectly (such as via an applicator or via a wipe or nonwoven carrier that includes the composition), topically or orally to the body or another surface. Depending upon the particular function, compositions of present invention may be rinsed with water or rubbed onto the skin and allowed to remain without rinsing. Preferably, the compositions of the present invention are rinse-off formulations, by which is meant the product is applied to a body surface and subsequently (i.e., within minutes) the skin or hair is rinsed with water, or otherwise wiped off using a substrate or other suitable removal means with deposition of a portion of the composition. Particularly suitable uses for compositions of the present invention include body washes and conditioners as well as hair shampoos and conditioners, and facial cleansers. Compositions of the present invention may also be used for cleansers with acne-treatment benefit agents, oral care rinses, eye treatments, among other personal care applications.

The compositions produced via the present invention may be preferably used as or in personal care products for treating or cleansing at least a portion of the human body. Examples of certain preferred personal care products include various products suitable for application to the skin, hair, and/or vaginal region of the body, such as shampoos, hand, face, and/or body washes, bath additives, gels, lotions, creams, and the like. As discussed above, applicants have discovered unexpectedly that the instant methods provide cost-effective personal care products having good aesthetics, and in certain embodiments one or more of desirable properties such as foaming characteristics, rheology, foam, and high surfactant loading.

The composition may be used to treat or cleanse the oral cavity, including oral mucosa and/or teeth.

The present invention provides methods of treating and/or cleansing the human body comprising contacting at least a portion of the body with a composition of the present invention. Certain preferred methods comprising contacting mammalian skin, hair and/or vaginal region with a composition of the present invention to cleanse such region and/or treat such region for any of a variety of conditions including, but not limited to, acne, wrinkles, dermatitis, dryness, muscle pain, itch, and the like. In certain preferred embodiments, the contacting step comprises applying a composition of the present invention to human skin, hair, oral cavity, eyes, or vaginal region.

The cleansing methods of the present invention may further comprise any of a variety of additional, optional steps associated conventionally with cleansing hair and skin including, for example, lathering, rinsing steps, and the like.

The present invention further provides methods of making a dilute structured composition comprising a branched fatty alcohol and a surfactant. While in certain embodiments, the order of mixing is not critical, it is preferable, in other embodiments, to pre-blend certain components, before adding such components to the others.

For example, in one particularly preferred embodiment, one or more structured concentrates may be formed by combining a branched fatty alcohol and a surfactant as well as a vehicle such as water among other optional ingredients. The structured concentrate may have a concentration of water that is, for example, from about 20% to about 70%.

The structured concentrate may be formed by blending water, surfactant (e.g., including betaine and branched anionic surfactant), and branched fatty alcohol by pouring, mixing, adding dropwise, pipetting, pumping, and the like, any one or more of such ingredients or compositions comprising such ingredients into any one or more of the other ingredients or compositions comprising such other ingredients in any order and optionally using any conventional equipment such as a mechanically stirred propeller, paddle, and the like. The structured concentrate may have a Yield Stress that is from about 1 Pascal (Pa) to about 10,000 Pa. In certain preferred embodiments the structured concentrate has a Yield Stress of from about 1 Pa to about 1500 Pa, and, more preferably those having a Yield Stress of from about 10 Pa to about 1100 Pa, as measured by the Yield Stress Method described hereafter.

The structured concentrate may be then combined with a sufficient amount of water to raise the concentration of water to at least about 80% to form a dilute structured composition. The pH may be optionally adjusted with a pH adjuster, such as citric acid or sodium hydroxide, to render the dilute structured composition with a pH greater than about 7.

The methods of the present invention may further comprise any of a variety of steps for mixing or introducing one or more of the optional components described hereinabove with or into the dilute structured composition of the present invention either before, after, or simultaneously with the combining step described above.

### EXAMPLES

The following Yield Stress Test is used in the instant methods and in the following Examples. In particular, as described above, the Yield Stress test is used to determine whether a structured concentrate is structured, according to the present invention.

### Yield Stress Test:

The following Yield Stress Test is performed on various personal care compositions to determine the Yield Stress according to the present invention. Samples are placed in a water bath set at 25° C for a period time sufficient to allow the sample to equilibrate (at least about an hour). The procedure is accomplished by gently placing about 1.0 grams of the composition to be tested was on the base plate of a properly calibrated rheometer (e.g., Advanced Rheometer AR 2000) having a 20 mm cone with a 1 degree angle, a 20 mm plate, a water bath, and a solvent trap. The sample size is just sufficient to allow some minor flow of the sample out of the gap once the final position of the cone and plate was reached (0.030 mm). To minimize shearing of the sample prior to testing, each sample is applied to the plate in a consistent manner, by gently scooping out the sample in one motion without significant shear or spreading, evenly layered on the plate, and without compressing and rotating the spatula away from the sample. The sample is centered on the base plate and laid relatively even across the plate. Once the measurement position is reached, a small bulge of the sample material protrudes from the gap. This is removed quickly and gently so as not to disturb the top plate and pre-shear the sample. [If the top plate was moved then the run is aborted.] The sample preparation described thus far is less than 20 seconds to reduce undue drying of the sample. The instrument was set for a controlled shear rate run (log) with a shear rate spanning from 0.01·⁻¹, to 300·⁻¹; 300 data points collected; 300 seconds test duration ; 25°C water bath. The output device attached to the rheometer is set to plot stress (Pa) as a function of shear rate s⁻¹. Yield stress is determined from the plot of yield stress versus shear rate as the stress at which the curve departs from linearity. The average and standard deviation of the 3 runs is determined.

### Dynamic Light Scattering Test ("DLS Test"):

Dynamic light scattering (DLS, also known as Photon Correlation Spectroscopy or PCS) is a well-known method for determination of average micelle and vesicle size (measured as hydrodynamic diameter, *d*_{H}) and their size distributions (A comprehensive explanation of the technique can be found in the ISO test method ISO13321:1996(E). The hydrodynamic size measured by DLS is defined as the size of a hypothetical hard sphere that diffuses in the same fashion as that of the particles being measured. In practice, micellar and vesicle species are dynamic (tumbling), solvated species that maybe isotropic (spherical) or anisotropic (e.g. ellipsoidal or cylindrical) in shape. Because of this, the diameter calculated from the diffusional properties of the particle will be indicative of the *apparent* size of the dynamic hydrated/solvated particle; hence the terminology, "hydrodynamic diameter." Solutions for the determination of particle *d*_{H} are prepared by diluting the compositions to either 3.0% of their original concentration with 0.1 µm-filtered water, obtained from a Millipore-Q filtration system. (The target dilution of 3.0% is chosen because it is within the typical concentration range of 1.0% - 10% dilution that is encountered during the use of rinse-off personal care compositions.) The samples are agitated on a vortex mixer at 1000 rpm for a minimum of five minutes and then allowed to stand overnight prior to analysis.

The samples are analyzed using a Zetasizer Nano ZS DLS instrument (Malvern Instruments, Inc., Southborough, MA) operating at 25.0°C. Samples must yield a minimum count rate of 100,000 counts per second (cps) for accurate determination of micelle and vesicle *d*_{H} and particle size distribution. The diluted samples were room temperature when loaded into the cuvette and then heated to and held at the target temperature (25.0 °C) for 30 mins before the measurement began. Values of micelle and vesicle *d*_{H} and their size distributions are calculated using the Dispersion Technology Software (DTS) v4.10 package (Malvern Instruments Inc., Southborough, MA), which for a system with different particle species calculates the particle *d*_{H} according to the nonlinear least squares fitting method to separate out the peak(s) that are generated from the micelles and vesicles. The reported values of the particle *d*_{H} are the average of three individual measurement runs.

Using the Dispersion Technology Software (DTS), the percent of total scattering intensity that was related to structures having size of 100 nm and above, i.e., the "DLS₁₀₀₊" is determined by integrating the area under the intensity spectrum for all hydrodynamic diameters 100 nm and above. Similary "DLS₂₀₀₊" is the percent of total intensity that was related to structures having size of 200 nm and above and is determined by integrating the area under the intensity spectrum for all hydrodynamic diameters 200 nm and above.

### Example Ex.1: Preparation of Inventive Example

The structured concentrate of Examples Ex.1 was prepared by blending a particular ingredient with other ingredients according to the materials and amounts listed in Table 1:

**Table 1**

| **Trade Name** | **INCI Name** | **Wt. %** |
|---|---|---|
| Water | Water | 30.84 |
| Emery 917 | Glycerin | 3.00 |
| Tegobetaine L-7V | Cocamidopropyl Betaine | 30.00 |
| Versene NA | Disodium EDTA | 0.30 |
| Miranol Ultra L-32 | Sodium Lauroamphoacetate | 6.00 |
| Isalchem 123 A | C12-13 Alcohol | 3.00 |
| Cedapal TD403 | Sodium Trideceth Sulfate | 26.00 |
| Glydant Plus Liquid | DMDM Hydantoin & Iodopropynyl Butylcarbamate | 0.40 |
| Citric Acid (50 % Solution) | Citric Acid | 0.46 |
| | | 100.00 |

The structured concentrate noted in Table 1 was prepared as follows: Ingredients were added in the order listed to a suitable size vessel equipped with an overhead propeller type mixer. Tegobetaine L-7V is 32% active betaine by weight. Miranol Ultra L-32 is 32% active sodium lauroamphoacetate by weight. Cedepal TD403 is 31 % sodium trideceth sulfate by weight.

Agitation was sufficient to maintain good batch movement without aeration. Components were added while maintaining constant agitation. pH was measured after the last component was added and adjusted to 5.5 - 6.5 Citric acid was then added to reduce to pH to between about 5.7. The structured concentrate shown in Table 1 was diluted with deionized water by adding 97 parts by weight of water to 3 parts by weight of the structured composition shown in Table 1. The dilution had a pH of about 7.0 and required no further adjustment in pH. To make a sample at pH 9, sufficient sodium hydroxide was added to raise the pH of the dilution to 9. The resulting dilute structured composition, Ex. 1 was as follows:

**Table 2**

| **Trade Name** | **INCI Name** | **Wt. %** |
|---|---|---|
| Water | Water | 97.93 |
| Emery 917 | Glycerin | 0.09 |
| Tegobetaine L-7V | Cocamidopropyl Betaine | 0.90 |
| Versene NA | Disodium EDTA | 0.01 |
| Miranol Ultra L-32 | Sodium Lauroamphoacetate | 0.18 |
| Isalchem 123 A | C12-13 Alcohol | 0.09 |
| Cedapal TD403 | Sodium Trideceth Sulfate | 0.78 |
| Glydant Plus Liquid | DMDM Hydantoin & lodopropynyl Butylcarbamate | 0.01 |
| Citric Acid (50 % Solution) | Citric Acid | 0.01 |
| | | 100.00 |

Converting the above concentrations to "active" (by accounting for water present in Tegobetaine, Cedepal, and Miranol) there is about 0.29% active betaine, 0.058% active amphoacetate, 0.24% trideeth sulfate, and about 99% water in Inventive Example, Ex. 1.

### Comparative Examples Comp. 1-2: Preparation of Comparative Examples

A structured concentrate was prepared by blending a particular ingredient with other ingredients according to the materials and amounts listed in Table 3:

**Table 3**

| **Item** | **INCI Name** | **Trade Name** | **Ingredient Wt%** | **Active Wt%** |
|---|---|---|---|---|
| 1 | Purified Water | Purified Water | 41.67 | 79.00 |
| 2 | Soidum Lauroamphoacetate (31%) | Miranol Ultra L-32 | 22.24 | 6.89 |
| 3 | Sodium Trideceth Sulfate (29.5%) | Rhodapex EST-30 | 31.01 | 9.15 |
| 4 | Cocamide MEA (97%) | Alkamide CME | 4.08 | 3.96 |
| 5 | Sodium Chloride | Sodium Chloride | 1.00 | 1.00 |
| 6 | Citric Acid | Citric Acid Solution, 20% | Q.S. | Q.S. |
| 7 | Sodium Hydroxide | Sodium Hydroxide Solution, 20% | Q.S. | Q.S. |
| 8 | Purified Water | Purified Water | Q.S. | Q.S. |
| | | | **100.00** | **100.00** |

The structured concentrate noted in Table 3 was prepared as follows: Ingredients were added in the order listed to a suitable size vessel equipped with an overhead propeller type mixer. Agitation was sufficient to maintain good batch movement without aeration. Components were added while maintaining constant agitation. After adding Rhodapex, the mixture was brought to 60C and Alkamide was added. The mixture was then cooled to 30C and the remaining ingredients were added. pH was measured after the last component was added and adjusted to 5.5 - 6.5 Citric acid was then added to reduce to pH to between about 5 - 6. The composition of Table 3 is similar to structured concentrates described in published U.S. patent application, US20030180246. Comp. 1, was completed by taking the structured concentrate of Table 3 and diluting it. Specifically, 3 parts by weight of the structured concentrate of Table 3 was added to 97 parts by weight of deionized water. Sufficient sodium hydroxide was added to raise the pH of the dilution to 7 or 9.

Similarly, a structured concentrate was prepared by blending a particular ingredient with other ingredients according to the materials and amounts listed in Table 4:

**Table 4**

| **Item** | **INCI Name** | **Trade Name** | **Ingredient Wt%** | **Active Wt%** |
|---|---|---|---|---|
| 1 | Purified Water | Purified Water | 14.49 | 71.80 |
| 2 | Soidum Lauroamphoacetate (31%) | Miranol Ultra L-32 | 48.38 | 15.00 |
| 3 | Sodium Trideceth Sulfate (29.5%) | Rhodapex EST-30 | 33.90 | 10.00 |
| 4 | Lauric Acid (99%) | Kortacid 1299 | 3.23 | 3.20 |
| 5 | Citric Acid | Citric Acid Solution, 20% | Q.S. | Q.S. |
| 6 | Sodium Hydroxide | Sodium Hydroxide Solution, 20% | Q.S. | Q.S. |
| 7 | Purified Water | Purified Water | Q.S. | Q.S. |
| | | | **100.00** | **100.00** |

The structured concentrate noted in Table 4 was prepared similarly to the composition described in Table 3. After adding Rhodapex, the mixture was brought to 50C and Kortacid was added. The mixture was then cooled to 30C and the remaining ingredients were added. pH was measured after the last component was added and adjusted to 5.3 - 5.7 using citric acid or sodium hydroxide. The composition of Table 4 is similar to structured surfactant compositions described in US patent, 6,150,312. Comp. 2 was completed by taking the structured concentrate of Table 4 and diluting it. Specifically, 3 parts by weight of the structured concentrate of Table 4 was added to 97 parts by weight of deionized water. Sufficient sodium hydroxide was added to raise the pH of the dilution to 7 or 9.

### Example 3: Evaluation of Structuring

Six experiments were conducted to compare the ability of 3 compositions to maintain their structuring upon dilution with water to 2 different pHs. Compositions Ex. 1, Comp. 1 and Comp. 2 diluted to pH 7 and pH 9 were evaluated according to the DLS Test. The percent of total intensity that was related to structures having size of 100nm and above is reported in Table 5 below. Similarly, the percent of total intensity that was related to structures having size of 200nm and above is also reported. It can be seen from the Table that Inventive Example, Ex. 1 has a much larger percent of its structures are larger (e.g., above 100nm or above 200nm) as compared to the comparative examples, Comp. 1 and Comp. 2. This suggests that only Inventive Example, Ex. 1 was able to maintain large (e.g., lamellar) structures, when subject to both dilution and to relatively high pH. By contrast, the comparative examples, the large structures present in the structured concentrates that were used to form Comp. 1 and Comp. 2 essentially disintegrated, leaving primarily smaller structures (e.g., surfactant micelles).

| **Composition** | **PH** | **Minimum Size (nm)** | **Intensity (%)** |
|---|---|---|---|
| Ex. 1 | 7 | 100 | 95.3 |
| Comp. 1 | 7 | 100 | 76.6 |
| Comp. 2 | 7 | 100 | 29.7 |
| Ex. 1 | 7 | 200 | 66.8 |
| Comp. 1 | 7 | 200 | 47.3 |
| Comp. 2 | 7 | 200 | 3.6 |
| Ex. 1 | 9 | 100 | 83.2 |
| Comp. 1 | 9 | 100 | 8.6 |
| Comp. 2 | 9 | 100 | 4.8 |
| Ex. 1 | 9 | 200 | 18.7 |
| Comp. 1 | 9 | 200 | 4.4 |
| Comp. 2 | 9 | 200 | 4.8 |

## Claims

1. A structured composition comprising a branched fatty alcohol, a surfactant, and at least about 80% water, wherein said composition has a pH of at least about 7, and wherein said composition has DLS₁₀₀₊ intensity of at least about 80%.

2. The structured composition of claim 1, wherein said composition has DLS₁₀₀₊ intensity of at least about 90%; or at least about 50%; or at least about 60%.

3. The structured composition of claim 1 or claim 2, wherein said surfactant is an anionic surfactant or amphoteric surfactant.

4. The structured composition of claim 3, wherein said surfactant is a betaine.

5. The structured composition of claim 3, wherein said surfactant is a branched anionic surfactant.

6. The composition of any preceding claim, wherein said branched fatty alcohol is monobranched.

7. The composition of claim 6, wherein said branched fatty alcohol is a monobranched, primary alcohol, preferably a monobranched, primary alcohol having from about 7 to about 22 carbon atoms.

8. The composition of claim 5, wherein said branched anionic surfactant is selected from the group consisting of a tridecyl sulfate, sodium C₁₂₋₁₃ alkyl sulfate, a C₁₂₋₁₅ alkyl sulfate, a C₁₂₋₁₅ alkyl sulfate, sodium C₁₂₋₁₈ alkyl sulfate, a C₁₀₋₁₆ alkyl sulfate, a trideceth sulfate, a C₁₂₋₁₃ pareth sulfate, a C₁₂₋₁₃ pareth-n sulfate, a C₁₂₋₁₄ pareth-n sulfate, and combinations thereof.

9. The composition of claim 5, wherein said branched anionic surfactant is a trideceth sulfate.

10. The composition of claim 4, wherein said betaine is an amidoalkyl betaine.

11. The composition of any preceding claim, further comprising an associative thickener.

12. The composition of any preceding claim, wherein the composition comprises at least about 90% water.

13. A personal care product comprising a composition of any one of claims 1 to 12.

14. The personal care product of claim 13 wherein said product is selected from the group consisting of shampoos, soaps, washes, bath additives, gels, lotions, creams, and oral care products suitable for topical or oral application.

15. A method of making a dilute structured composition, comprising:
combining a branched fatty alcohol and water to form a structured concentrate having a Yield Stress from about 1 Pascal (Pa) to about 1500 Pa; and
adding sufficient additional water to form a dilute structured composition having at least about 80% water.
